# FASCICULE DE BREVET EUROPEEN

(11) **EP 1 084 698 B1**
(45) Date de publication et mention de la délivrance du brevet: **09.07.2003**
(21) Numéro de dépôt: 00402377.6
(22) Date de dépôt: 28.08.2000
(51) Int. Cl.: A61K 7/11, A61K 7/06

(54) **Composition cosmétique comprenant au moins un copolymère silicone/acrylate et au moins un polymère siliconé greffé**
Kosmetische Zusammensetzung, die mindestens ein Silikon/Acrylat-Copolymer und mindestens ein gepfropftes Silikonpolymer enthält
Cosmetic composition containing at least a silicone/acrylate copolymer and at least a silicone graft copolymer

(30) Priorité: 16.09.1999 FR 9911596
(43) Date de publication de la demande: 21.03.2001
(73) Titulaire: L'OREAL, 75008 Paris (FR)
(72) Inventeur: Dupuis, Christine, 75008 Paris (FR)
(74) Mandataire: Bourdeau, Françoise

(56) Documents cités:
- EP-A- 0 408 311
- EP-A- 0 749 747
- WO-A-99/04750
- FR-A- 2 740 037

## Description

L'invention a pour objet une composition cosmétique, comprenant au moins un copolymère silicone/acrylate particulier et au moins un polymère siliconé greffé, à squelette polysiloxanique greffé par des monomères organiques non siliconés. Elle vise également un procédé cosmétique, en particulier un procédé de fixation et/ou de maintien de la coiffure mettant en oeuvre ladite composition ainsi que l'utilisation de cette composition pour la fabrication d'une formulation cosmétique, destinée notamment au maintien et/ou à la mise en forme de la coiffure.

Parmi les produits capillaires, notamment ceux destinés à la mise en forme et/ou au maintien de la coiffure les plus répandus sur le marché de la cosmétique, on distingue les compositions à pulvériser essentiellement constituées d'une solution le plus souvent alcoolique ou aqueuse et d'un ou plusieurs matériaux.

Lorsque les compositions sont destinées à la fixation et/ou au maintien de la coiffure, ces matériaux sont généralement des résines polymères, dont la fonction est de former des soudures entre les cheveux, appelés encore matériaux fixants, en mélange avec divers adjuvants cosmétiques. Ces compositions sont généralement conditionnées soit dans un récipient aérosol approprié mis sous pression à l'aide d'un propulseur, soit dans un flacon pompe.

On connaît également les gels ou les mousses capillaires qui sont généralement appliqués sur les cheveux mouillés avant de faire un brushing ou une mise en plis. Pour mettre en forme et fixer la coiffure, on effectue ensuite un brushing ou un séchage. Ces gels ou mousses peuvent également contenir des résines polymères.

Toutefois, ces compositions capillaires présentent souvent l'inconvénient d'altérer les propriétés cosmétiques des cheveux. Ainsi, les cheveux peuvent devenir rêches, difficiles à démêler, perdre leur toucher et leur aspect agréables ou encore manquer de corps. On recherche donc des compositions coiffantes procurant de bonnes propriétés cosmétiques, notamment en terme de démêlage, de douceur et de toucher.

En outre, ces compositions capillaires présentent également l'inconvénient majeur de donner lieu à une effet de poudrage. Au sens de la présente invention, on entend par « poudrage », l'aptitude du matériau obtenu par séchage de la composition capillaire à former une poudre après son application sur les cheveux. Bien entendu, la poudre tombe sur les épaules ou les vêtements de l'utilisateur, ou encore elle accroche au peigne ou à la brosse, et ceci est préjudiciable.

Il existe donc un besoin de trouver des compositions cosmétiques, notamment pour le coiffage, qui ne présentent pas l'ensemble des inconvénients indiqués ci-dessus, et qui en particulier fixent bien la coiffure, tout en procurant de bonnes propriétés cosmétiques, ceci sans effet de poudrage.

De manière surprenante et inattendue, la Demanderesse a découvert que lorsque l'on associe des copolymères silicone/acrylate particuliers avec certains polymères particuliers, il est possible d'obtenir des compositions cosmétiques répondant aux exigences exprimées ci-dessus.

L'invention a pour objet une composition cosmétique comprenant, dans un milieu cosmétiquement acceptable, au moins un copolymère silicone/acrylate et au moins un polymère siliconé greffé, à squelette polysiloxanique greffé par des monomères organiques non-siliconés, le copolymère silicone/acrylate étant obtenu par polymérisation radicalaire d'au moins un monomère éthyléniquement insaturé (a) en présence d'au moins un dérivé siliconé (b) comprenant des groupements oxyalkylénés.

Un autre objet de l'invention concerne un procédé cosmétique capillaire, en particulier un procédé de fixation et/ou de maintien de la coiffure mettant en oeuvre ladite composition.

Encore un autre objet de l'invention concerne l'utilisation de cette composition pour la fabrication d'une formulation cosmétique capillaire, destinée notamment au maintien et/ou à la mise en forme de la coiffure.

Les copolymères silicone/acrylate particulièrement visés par la présente invention sont ceux décrits dans la demande internationale WO99/04750. En particulier, on préfère le copolymère commercialisé par BASF sous la dénomination Luviflex Silk. Ce polymère un copolymère greffé acrylate de tertiobutyle/ acide méthacrylique et silicone copolyol.

De préférence, le monomère (a) répond à la formule (Iₐ) : 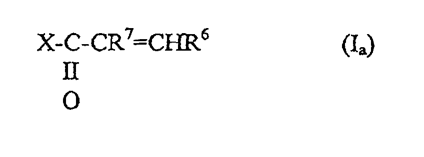 dans laquelle :
- X est choisi dans le groupe comprenant OH, OM, OR⁸, NH₂, NHR⁸, N(R⁸)₂ ;
- M est un cation choisi parmi Na⁺, K⁺, Mg⁺⁺, NH ⁴⁺ , alkylammonium, dialkylammonium, trialkylammonium, et tétraalkylammonium ;
- les radicaux R⁸ peuvent être identiques ou différents et sont choisis dans le groupe comprenant l'hydrogène, les groupements alkyls linéaires ou ramifiés en C₁ à C₄₀, les groupements alkyls en C₁ à C₄₀ mono- ou polyhydroxylés, éventuellement substitués par un ou plusieurs groupement(s) alcoxy, amino ou carboxy, les groupements polyéthers hydroxylés, les groupements N,N-diméthylaminoéthyl, 2-hydroxyéthyl, 2-méthoxyéthyl, 2-éthoxyéthyl, hydroxypropyl, méthoxypropyl et éthoxypropyl ;
- R⁷ et R⁶ sont choisis indépendamment l'un de l'autre dans le groupe comprenant l'hydrogène, les groupements alkyls linéaires ou ramifiés en C₁ à C₈, méthoxy, éthoxy, 2-hydroxyéthoxy, 2-méthoxyéthoxy et 2-éthoxyéthyl, les groupements CN, COOH et COOM.

En particulier, les monomères (a) de formule (Ia) sont l'acide acrylique et ses sels, esters ou amides. Les esters peuvent être dérivés d'alkyles linéaires en C₁ à C₄₀, d'alkyles ramifiés en C₃ à C₄₀ ou d'alcools carboxyliques en C₃ à C₄₀, d'alcools polyfonctionnels ayant 2 à 8 hydroxyles, tel que l'éthylène glycol, l'hexylène glycol, le glycérol et le 1,2,6-hexanetriol, d'aminoalcools ou d'éthers d'alcools tels que le méthoxyméthanol et l'éthoxyéthanol ou les polyalkylènes glycols.

Les monomères (a) de formule (Ia) peuvent également être choisis parmi les N,N-dialkylaminoalkyl acrylates et méthacrylates et N-dialkylaminoalkylacryl- et -méthacrylamides, le groupement amide pouvant être non substitué, N-aklyl- ou N-alkylamino-monosubstitué ou N,N-dialkylamino-disubstitué, les groupes alkyles ou alkylamino étant dérivés d'unités carboxyliques linéaires en C₁ à C₄₀ ou ramifiées en C₃ à C₄₀.

Les monomères (a) de formule (la) pouvant être utilisés peuvent aussi être des composés substitués dérivés de l'acide acrylique ou ses sels, esters ou amides. On peut citer par exemple les acides méthacrylique, éthacrylique et 3-cyanoacrylique.

D'autres monomères (a) convenant particulièrement bien sont les esters de vinyle et d'allyle en C₁ à C₄₀, les acides carboxyliques, linéaires en C₃ à C₄₀, ou carboxycycliques en C₃ à C₄₀, les halides de vinyle ou d'allyle, les vinyllactames, de préférence la vinylpyrrolidone et le vinylcaprolactame, les composés hétérocycles substitués par des groupement vinyles ou allyles, de préférence la vinylpyridine, la vinyloxazoline et l'allylpyridine, les N-vinylimidazoles, les diallylamines, le chlorure de vinylidène, les composés insaturés carbonés, comme le styrène ou l'isoprène, les dérivés quaternisés par l'épichlorhydrine de l'acide acrylique ou méthacrylique

Comme autre monomère (a), on peut enfin citer les N-vinylimidazoles, les diallylamines, le chlorure de vinylidène, les composés insaturés carbonés comme le styrène et l'isoprène, les dérivés quaternisés par l'épichlorohydrine de l'acide acrylique ou méthacrylique.

On préfère tout particulièrement, comme monomère (a) l'acide acrylique, méthacrylique et éthylacrylique; le méthyl, éthyl, propyl, n-butyl, isobutyl, t-butyl, 2-éthylhexyl et décyl acrylate ; le méthyl, éthyl, propyl, n-butyl, isobutyl, t-butyl, 2-éthylhexyl et décyl méthacrylate ; le méthyl, éthyl, propyl, n-butyl, isobutyl, t-butyl, 2-éthylhexyl et décyl éthacrylate ; le 2,3-dihydroxypropyl acrylate ; le 2,3-dihydroxypropyl méthacrylate ; le 2-dihydroxyéthyl acrylate ; l'hydroxypropyl acrylate ; le 2-hydroxyéthyl méthacrylate, le 2-hydroxyéthyl éthacrylate ; le 2-méthoxyéthyl acrylate; le 2-éthoxyéthyl méthacrylate; le 2-éthoxyéthyl éthacrylate ; l'hydroxypropyl méthacrylate ; le glycéryl monoacrylate; le glycéryl monométhacrylate ; les polyalkylènes glycols( méth-) acrylate, les acides sulfoniques insaturés, l'acrylamide, la méthacrylamide, l'éthacrylamide, la N,N-diméthylacrylamide, la N-éthylacrylamide, la N-5 éthylméthacrylamide, 1-vinylimidazole, N,N-diméthylaminoéthyl(méth-)acrylate, l'acide maléique, l'acide fumarique, l'anhydride maléique et ses monoesters, l'acide crotonique, l'acide itaconique, les éthers de vinyle, le vinylformamide, la vinylamine, la vinylpyridine, la vinylimidazole, le vinylfurane, le styrène, le sulfonate de styrène, l'alcool allylique et leurs mélanges.

Le monomère (a) peut contenir également des atomes de silicium, de fluor ou encore des groupements thio. Les monomères (a) peuvent être neutralisés s'ils contiennent des groupement acides, et ceci avant ou après la polymérisation, totalement ou partiellement, de façon à ajuster la solubilité ou le degré de dispersion dans l'eau au niveau désiré. En tant qu'agent servant pour la neutralisation, on peut utiliser les bases minérales, telles que le carbonate de sodium, les bases oraganiques telles que les aminoalcools, comme les alcanolamines telles que la méthanolamine, le 2-amino-2-méthyl-1-propanol, la triéthanolamine, les diamines, comme la lysine.

Les monomères (a) peuvent être aussi quaternisés lorsqu'ils possèdent un atome d'azote basique. S'ils possèdent au moins deux doubles liaisons éthyléniques, les monomères (a) peuvent être partiellement réticulés.

Les dérivés siliconés (b) sont notamment les composés connus, sous la dénomination INCI par diméthicone copolyols ou les tensioactifs siliconés. On peut citer ceux commercialisés sous la marque Abil ® par Goldschmidt, Alkasil ® par Rhône-Poulenc, silicone Polyol Copolymer ® par Genesee, Besil ® par Wacker, Silwet ® par OSI ou Dow Corning 190® par Dow Corning.

Parmi les monomères (b), on préfère ceux qui présentent la formule I ci-après : 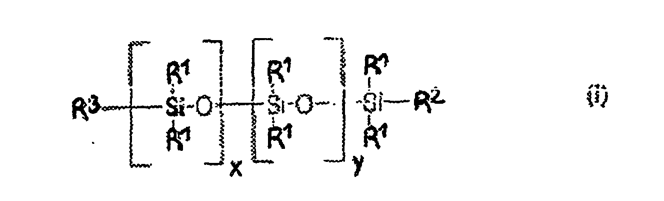 dans laquelle formule (I):
- R² est choisi parmi CH₃ ou le groupement 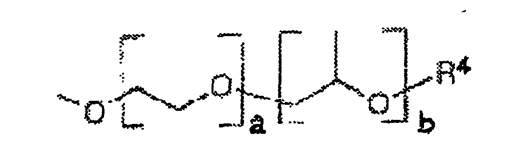
- R³ est choisi parmi CH₃ ou le groupement R²,
- R⁴ est choisi parmi l'hydrogène, CH₃, ou les groupements 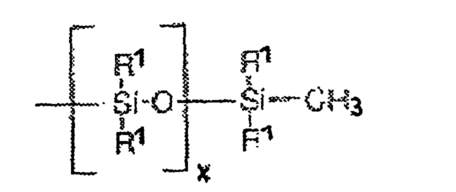 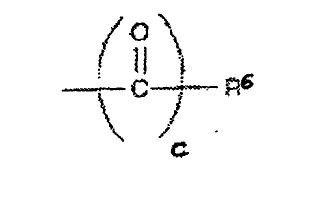
- R⁶ est un groupement organique en C₁ à C₄₀ pouvant contenir des groupements amino, carboxyles ou sulfonates, et si c=0, R₆ est l'anion d'un acide inorganique ;
- les radicaux R¹ peuvent être identiques ou différents et sont choisis parmi les hydrocarbures aliphatiques en C₁ à C₂₀, les hydrocarbures aliphatiques ou cycloaliphatiques en C₃ à C₂₀, et les groupements R⁵ répondant à la formule ci-après : 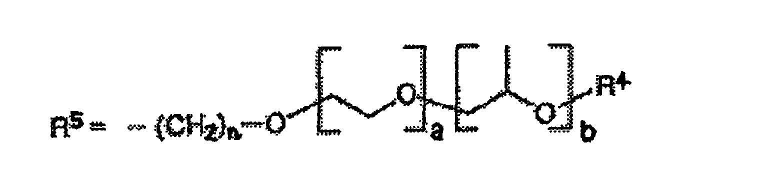
- n est un nombre entier compris entre 1 et 6,
- x et y sont des nombres entiers choisis de telle sorte que le poids moléculaire du polysiloxane soit compris entre 300 et 30 000,
- a et b sont des nombres entiers compris entre 0 et 50, et
- c est 0 ou 1.

De préférence, R¹ est choisi parmi les groupements méthyl, éthyl, propyl, butyl, isobutyl, pentyl, isopentyl, hexyl, octyl, décyl, dodécyl and octadécyl, les radicaux cycloaliphatiques, en particulier les groupements cyclohexyls, les groupements aromatiques, en particulier les groupements phényls ou naphthyls, les mélanges de radicaux aromatiques et aliphatiques, tels que le benzyl et le phenyléthyl, et aussi le tolyl and le xylyl.

On préfère les radicaux R⁴ ayant pour formule -(CO)c-R⁶, R⁶ étant un alkyl, cycloalkyl or aryl ayant 1 à 40 atomes de carbone qui peut contenir des groupements supplémentaires tels que NH₂, COOH et/ou SO₃H.

On préfère les radicaux R⁶ pour lesquels c=0 qui sont des phosphates ou des sulfates.

Les dérivés siliconés (b) particulièrement préférés sont ceux présentant la formule générale suivante :

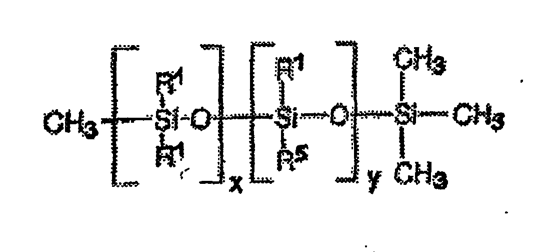

La proportion relative de dérivé (b) dans le copolymère est généralement de 0,1 à 50, et de préférence de 1 à 20 % en poids.

On préfère les copolymères silicone/acrylate solubles dans l'eau ou ceux dont la dispersibilité dans l'eau est telle que dans un mélange eau/éthanol dosé à 50 :50 en volume, ils sont solubles dans une proportion supérieure à 0,1 g/l, et de préférence supérieure à 0,2 g/l.

La composition comprend avantageusement, en pourcentage relatif en poids de la composition, de 0,1 à 20 % de polymère siliconé greffé, à squelette polysiloxanique greffé par des monomères organique non-siliconé, et de préférence, de 0,2 à 10 %.

La composition comprend avantageusement en pourcentage relatif en poids, entre 0,1 et 20 % de copolymère silicone/acrylate, et plus préférentiellement entre 0,5 et 10 % de ce polymère.

Dans ce qui suit, on entend désigner par polymère siliconé, en conformité avec l'acceptation générale, tous polymères ou oligomères organosiliciés à structure linéaire ou cyclique, ramifiée ou réticulée, de poids moléculaire variable, obtenus par polymérisation et/ou polycondensation de silanes convenablement fonctionnalisés, et constitués pour l'essentiel par une répétition de motifs principaux dans lesquels les atomes de silicium sont reliés entre eux par des atomes d'oxygène (liaison siloxane ≡Si-O-Si≡), des radicaux hydrocarbonés éventuellement substitués étant directement liés par l'intermédiaire d'un atome de carbone sur lesdits atomes de silicium. Les radicaux hydrocarbonés les plus courants sont les radicaux alkyls notamment en C₁-C₁₀ et en particulier méthyle, les radicaux fluoroalkyls, les radicaux aryls et en particulier phényle, et les radicaux alcényls et en particulier vinyle ; d'autres types de radicaux susceptibles d'être liés soit directement; soit par l'intermédiaire d'un radical hydrocarboné, à la chaîne siloxanique sont notamment l'hydrogène, les halogènes et en particulier le chlore, le brome ou le fluor, les thiols, les radicaux alcoxy, les radicaux polyoxyalkylènes (ou polyéthers) et en particulier polyoxyéthylène et/ou polyoxypropylène, les radicaux hydroxyls ou hydroxyalkyls, les groupements aminés substitués ou non, les groupements amides, les radicaux acyloxy ou acyloxyalkyls, les radicaux hydroxyalkylamino ou aminoalkyls, des groupements ammonium quaternaires, des groupements amphotères ou bétaïniques, des groupements anioniques tels que carboxylates, thioglycolates, sulfosuccinates, thiosulfates, phosphates et sulfates, cette liste n'étant bien entendu nullement limitative (silicones dites "organomodifiées").

Selon la présente invention, le ou les polymères siliconés qui doivent être utilisés sont ceux qui comprennent une chaîne principale de silicone (ou polysiloxane (≡Si-O-)ₙ) sur laquelle se trouve greffé, à l'intérieur de ladite chaîne ainsi qu'éventuellement à l'une au moins de ses extrémités, au moins un groupement organique ne comportant pas de silicone.

Ces polymères siliconés peuvent être des produits commerciaux existants, ou encore être obtenus selon tout moyen connu de l'homme de l'art, en particulier par réaction entre (i) une silicone de départ correctement fonctionnalisée sur un ou plusieurs de ces atomes de silicium et (ii) un composé organique non-siliconé lui-même correctement fonctionnalisé par une fonction qui est capable de venir réagir avec le ou les groupements fonctionnels portés par ladite silicone en formant une liaison covalence ; un exemple classique d'une telle réaction est la réaction d'hydrosylilation entre des groupements ≡Si-H et des groupements vinyliques CH₂=CH-, ou encore la réaction entre des groupements thio-fonctionnels -SH avec ces mêmes groupements vinyliques.

Des exemples de polymères siliconés convenant à la mise en oeuvre de la présente invention, ainsi que leur mode particulier de préparation, sont notamment décrits dans les demandes de brevets EP-A-0582152, WO93/23009 et WO 95/03776.

Selon un mode particulièrement préféré de réalisation de la présente invention, le polymère siliconé mis en oeuvre comprend le résultat de la copolymérisation radicalaire entre d'une part au moins un monomère organique anionique non-siliconé présentant une insaturation éthylénique et/ou un monomère organique hydrophobe non-siliconé présentant une insaturation éthylénique et d'autre part une silicone présentant dans sa chaîne au moins un groupement fonctionnel capable de venir réagir sur lesdites insaturations éthyléniques desdits monomères non-siliconés en formant une liaison covalente, en particulier des groupements thio-fonctionnels.

Selon la présente invention, lesdits monomères anioniques à insaturation éthylénique sont de préférence choisis, seuls ou en mélanges, parmi les acides carboxyliques insaturés, linéaires ou ramifiés, éventuellement partiellement ou totalement neutralisés sous la forme d'un sel, ce ou ces acides carboxyliques insaturés pouvant être plus particulièrement l'acide acrylique, l'acide méthacrylique, l'acide maléique, l'anhydride maléique, l'acide itaconique, l'acide fumarique et l'acide crotonique. Les sels convenables sont notamment les sels d'alcalins, d'alcalino-terreux et d'ammonium. On notera que, de même, dans le polymère siliconé greffé final, le groupement organique à caractère anionique qui comprend le résultat de l'(homo)polymérisation radicalaire d'au moins un monomère anionique de type acide carboxylique insaturé peut être, après réaction, post-neutralisé avec une base (soude, ammoniaque,...) pour l'amener sous la forme d'un sel.

Selon la présente invention, les monomères hydrophobes à insaturation éthylénique sont de préférence choisis, seuls ou en mélanges, parmi les esters d'acide acrylique alcanols et/ou les esters d'acide méthacrylique d'alcanols. Les alcanols sont de préférence en C₁-C₁₈ et plus particulièrement en C₁-C₁₂. Les monomères préférentiels sont choisis dans le groupe constitué par le (méth)acrylate d'isooctyle, le (méth)acrylate d'isononyle, le 2-éthylhexyl(méth)acrylate, le (méth)acrylate de lauryle, le (méth)acrylate d'isopentyle, le (méth)acrylate de n-butyle, le (méth)acrylate d'isobutyle, le (méth)acrylate de méthyle, le (méth)acrylate de tertio-butyle, le (méth)acrylate de tridécyle, le (méth)acrylate de stéaryle ou leurs mélanges.

Une famille de polymères siliconés greffés particulièrement bien à la mise en oeuvre de la présente invention est constituée par les polymères siliconés comportant dans leur structure le motif de formule (I) suivant : 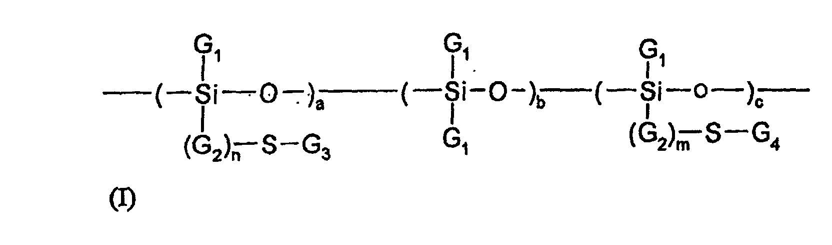 dans lequel les radicaux G₁ , identiques ou différents, représentent l'hydrogène ou un radical alkyle en C₁-C₁₀ ou encore un radical phényle ; les radicaux G₂, identiques ou différents, représentent un groupe alkylène en C₁-C₁₀ ; G₃ représente un reste polymérique résultant de l'(homo)polymérisation d'au moins un monomère anionique à insaturation éthylénique ; G₄ représente un reste polymérique résultant de l'(homo)polymérisation d'au moins un monomère hydrophobe à insaturation éthylénique ; m et n sont égaux à 0 ou 1 ; a est un nombre entier allant de 0 et 50 ; b est un nombre entier pouvant être compris entre 10 et 350, c est un nombre entier allant de 0 et 50 ; sous réserve que l'un des paramètres a et c soit différent de 0.

De préférence, le motif de formule (I) ci-dessus présente au moins l'une, et encore plus préférentiellement l'ensemble, des caractéristiques suivantes
- les radicaux G₁ désignent un radical alkyle, de préférence le radical méthyle ;
- n est non nul, et les radicaux G₂ représentent un radical divalent en C₁-C₃, de préférence un radical propylène ;
- G₃ représente un radical polymérique résultant de l'(homo)polymérisation d'au moins un monomère du type acide carboxylique à insaturation éthylénique, de préférence l'acide acrylique et/ou l'acide méthacrylique
- G₄ représente un radical polymérique résultant de l'(homo)polymérisation d'au moins un monomère du'type (méth)acrylate d'alkyle (C₁-C₁₀), de préférence le (méth)acrylate d'isobutyle ou de méthyle.

Des exemples de polymères siliconés répondant à la formule (I) sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth)acrylate de méthyle.

D'autres exemples de polymères siliconés répondant à la formule (I) sont notamment des polydiméthylsiloxanes (PDMS) sur lesquels sont greffés, par l'intermédiaire d'un chaînon de raccordement de type thiopropylène, des motifs polymères du type poly(méth)acrylate d'isobutyle.

De préférence, la masse moléculaire en nombre des polymères siliconés de l'invention varie de 10 000 à 1 000 000 environ, et encore plus préférentiellement de 10 000 à 100 000 environ.

Le milieu cosmétiquement acceptable est, de préférence, constitué par de l'eau ou un ou plusieurs solvants cosmétiquement acceptables tels que des alcools ou des mélanges eau-solvant(s), ces solvants étant de préférence des alcools en C₁-C₄.

Parmi ces alcools, on peut citer l'éthanol, l'isopropanol. L'éthanol est particulièrement préféré.

La composition de l'invention peut également contenir au moins un additif choisi parmi les tensioactifs anioniques, cationiques, non ioniques ou amphotères, les parfums, les filtres, les conservateurs, les protéines, les vitamines, les polymères autres que ceux de l'invention, les huiles végétales, minérales ou synthétiques et tout autre additif classiquement utilisé dans les compositions cosmétiques.

Bien entendu, l'homme de l'art veillera à choisir le ou les éventuels composés à ajouter à la composition selon l'invention de manière telle que les propriétés avantageuses attachées intrinsèquement à la composition conforme à l'invention ne soient pas, ou substantiellement pas, altérées par l'addition envisagée.

Ces compositions peuvent être conditionnées sous diverses formes, notamment dans des flacons pompes ou dans des récipients aérosols, afin d'assurer une application de la composition sous forme vaporisée ou sous forme de mousse. De telles formes de conditionnement sont indiquées, par exemple, lorsqu'on souhaite obtenir un spray, une laque ou une mousse pour la fixation ou le traitement des cheveux. Les compositions conformes à l'invention peuvent aussi se présenter sous la forme de crèmes, de gels, d'émulsions, de lotions ou de cires.

Lorsque la composition selon l'invention est conditionnée sous forme d'aérosol en vue d'obtenir une laque ou une mousse, elle comprend au moins un agent propulseur qui peut être choisi parmi les hydrocarbures volatils tels que le n-butane, le propane, l'isobutane, le pentane, un hydrocarbure halogéné et leurs mélanges. On peut également utiliser en tant qu'agent propulseur le gaz carbonique, le protoxyde d'azote, le diméthyléther (DME), l'azote, l'air comprimé. On peut aussi utiliser des mélanges de propulseurs. De préférence, on utilise le diméthyl éther.

Avantageusement, l'agent propulseur est présent à une concentration comprise entre 5 et 90 % en poids par rapport au poids total de la composition dans le dispositif aérosol et, plus particulièrement, à une concentration comprise entre 10 et 60 %.

Les compositions conformes à l'inventions peuvent être appliquées sur la peau, les ongles, les lèvres, les cheveux, les sourcils et les cils.

Les compositions conformes à l'invention sont particulièrement adaptés pour des cheveux secs ou humides, en tant que produits de coiffage.

L'invention va être plus complètement illustrée à l'aide des exemples non limitatifs suivants.

Tous les pourcentages sont des pourcentages relatifs en poids par rapport au poids total de la composition et m.a. signifie matière active.

### EXEMPLE

On prépare la composition suivante en flacon pompe.

| | |
|---|---|
| Luviflex Silk (BASF) ∗ | 4 % |
| Polysilicone 8 (VS80 commercialisé par la Société 3M) | 2 % |
| AMP qs | pH=7 |
| Eau | 14 % |
| Ethanol | qsp 100g |

| | |
|---|---|
| ∗ copolymère silicone/acrylate (t-butyl acrylate/acide méthacrylique/PDMS polyéther) | |

Cette composition est pulvérisée sur des cheveux eurochâtains. Après séchage, les cheveux sont bien maintenus et possèdent un toucher cosmétique.

## Revendications

1. Composition cosmétique comprenant, dans un milieu cosmétiquement acceptable, au moins un copolymère silicone/acrylate et au moins un polymère siliconé greffé, à squelette polysiloxanique greffé par des monomères organiques non-siliconés, **caractérise par le fait que** le copolymère silicone/acrylate est obtenu par polymérisation radicalaire d'au moins un monomère éthyléniquement insaturé (a) en présence d'au moins un dérivé siliconé (b) comprenant des groupements oxyalkylénés.

2. Composition selon la revendication 1, **caractérisée par le fait que** le monomère (a) répond à la formule (Iₐ): 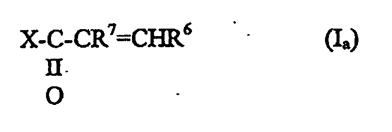 dans laquelle :
- X est choisi dans le groupe comprenant OH, OM, OR⁸, NH₂, NHR⁸, N(R⁸)₂;
- M est un cation choisi parmi Na⁺, K⁺, Mg⁺⁺, NH ⁴⁺ , alkylammonium, dialkylammonium, trialkylammonium, et tétraalkylammonium;
- les radicaux R⁸ peuvent être identiques ou différents et sont choisis dans le groupe comprenant l'hydrogène, les groupements alkyls linéaires ou -ramifiés en C₁ à C₄₀, les groupements alkyls en C₁ à C₄₀ mono- ou polyhydroxylés, éventuellement substitués par un ou plusieurs groupement(s) alcoxy, amino ou carboxy, les groupements polyéthers hydroxylés, le N,N-diméthylaminoéthyl, le 2-hydroxyéthyl, le 2-méthoxyéthyl, le 2-éthoxyéthyl, le hydroxypropyl, le méthoxypropyl et l'éthoxypropyl ;
- R⁷ et R⁶ sont choisis indépendamment l'un de l'autre dans le groupe comprenant l'hydrogène, les groupements alkyls linéaires ou ramifiés en C₁ à C₈, méthoxy, éthoxy, 2-hydroxyéthoxy, 2-méthoxyéthoxy et 2-éthoxyéthyl, les groupements CN, COOH et COOM.

3. Composition cosmétique capillaire selon la revendication 1, **caractérisée par le fait que** le dérivé siliconé (b) répond à la formule I ci-après : 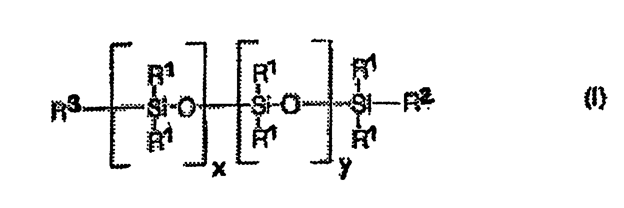 dans laquelle formule (I):
- R² est choisi parmi CH₃ ou le groupement 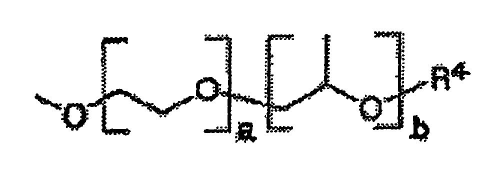
- R³ est choisi parmi CH₃ ou le groupement R²,
- R⁴ est choisi parmi l'hydrogène, CH₃, ou les groupements 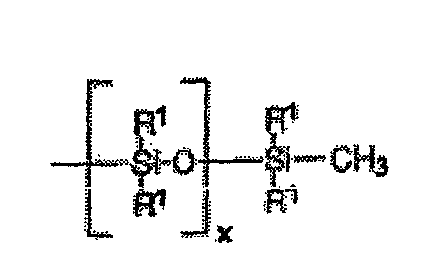 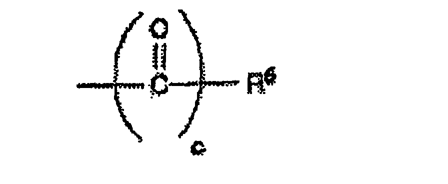
- R⁶ est un groupement organique en C₁ à C₄₀ pouvant contenir des groupements amino, carboxyles ou sulfonates, et si c=0, R₆ est l'anion d'un acide inorganique ;
- les radicaux R¹ peuvent être identiques ou différents et sont choisis parmi les hydrocarbures aliphatiques en C₁ à C₂₀, les hydrocarbures aliphatiques ou cycloaliphatiques en C₃ à C₂₀, et les groupements R⁵ répondant à la formule ci-après : 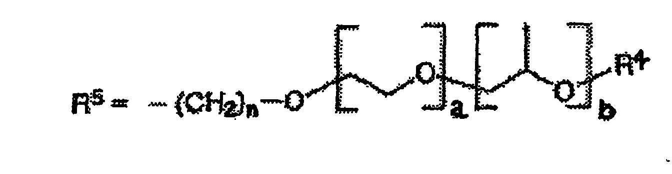
- n est un nombre entier compris entre 1 et 6,
- x et y sont des nombres entiers choisis de telle sorte que le poids moléculaire du polysiloxane soit compris entre 300 et 30 000,
- a et b sont des nombres entiers compris entre 0 et 50, et
- c est 0 ou 1,

4. Composition selon la revendication 1, **caractérisée par. le fait que** le monomère (a) est choisi parmi les esters de vinyle et d'allyle en C₁ à C₄₀, les acides carboxyliques, linéaires en C₃ à C₄₀, ou carboxycycliques en C₃ à C₄₀, les halides de vinyle ou d'allyle, les vinyllactames, de préférence la vinylpyrrolidone et le vinylcaprolactame, et les composés hétérocycles substitués par des groupement vinyls ou allyls, de préférence la vinylpyridine, la vinyloxazoline et l'allylpyridine, les N-vinylimidazoles, les diallylamines, le chlorure de vinylidène, les composés insaturés carbonés, comme le styrène ou l'isoprène, les dérivés quaternisés par l'épichlorhydrine de l'acide acrylique ou méthacrylique.

5. Composition selon la revendication 1, **caractérisée par le fait que** le monomère (a) est choisi dans le groupe comprenant l'acide acrylique, méthacrylique et éthylacrylique ; le méthyl, éthyl, propyl, n-butyl, isobutyl, t-butyl, 2-éthylhexyl et décyl acrylate ; le méthyl, éthyl, propyl, n-butyl, isobutyl, t-butyl, 2-éthylhexyl et décyl méthàcrylate ; le méthyl, éthyl, propyl, n-butyl, isobutyl, t-butyl, 2-éthylhexyl et décyl éthacrylate ; le 2,3-dihydroxypropyl acrylate ; le 2,3-dihydroxypropyl méthacrylate ; le 2-dihydroxyéthyl acrylate ; l'hydroxypropyl acrylate ; le 2-hydroxyéthyl méthacrylate ; le 2-hydroxyéthyl éthacrylate ; le 2-méthoxyéthyl acrylate ; le 2-éthoxyéthyl méthacrylate ; le 2-éthoxyéthyl éthacrylate ; l'hydroxypropyl méthacrylate; le glycéryl monoacrylate ; le glycéryl monométhacrylate ; les polyalkylènes glycols( méth-) acrylate, les acides sulfoniques insaturés, l'acrylamide, la méthacrylamide, l'éthacrylamide, la N,N-diméthylacrylamide, la N-éthylacrylamide, la N-éthylméthacrylamide, 1-vinylimidazole, N,N-diméthylaminoéthyl(méth-)acrylate, l'acide maléique, l'acide fumarique, l'anhydride maléique et ses monoesters, l'acide crotonique, l'acide itaconique, les éthers de vinyle, le vinylformamide, la vinylamine, la vinylpyridine, la vinylimidazole, le vinylfurane, le styrène, le sulfonate de styrène, l'alcool allylique et leurs mélanges.

6. Composition selon l'une quelconque des revendications précédentes, **caractérisée par le fait que** les dérivés siliconés (b) sont choisis parmi les diméthicone copolyols ou les tensioactifs siliconés.

7. Composition selon l'une quelconque des revendications précédentes, **caractérisée par le fait qu'**elle comprend, en pourcentage relatif en poids, de 0,1 à 20 % de copolymère silicone/acrylate, et plus préférentiellement de 0,5 à 10 % de ce copolymère.

8. Composition selon l'une quelconque des revendications précédentes, **caractérisée par le fait que** le polymère siliconé greffé comprend une chaîne principale de polysiloxane sur laquelle se trouve greffé, à l'intérieur de ladite chaîne ainsi qu'éventuellement à l'une au moins de ses extrémités, au moins un groupement organique ne comportant pas de silicone.

9. Composition selon l'une quelconque des revendications- précédentes, **caractérisée par le fait que** le polymère siliconé greffé est susceptible d'être obtenu par copolymérisation radicalaire entre d'une part au moins un monomère organique anionique non-siliconé présentant une insaturation éthylénique et/ou un monomère organique hydrophobe non-siliconé présentant une insaturation éthylénique et d'autre part un polysiloxane présentant dans sa chaîne au moins un groupement fonctionnel capable de venir réagir sur lesdites insaturations éthyléniques desdits monomères non-siliconés.

10. Composition selon la revendication 9, **caractérisée par le fait que** le monomère organique anionique à insaturation éthylénique est choisi, seul ou sous forme de mélange de monomères, parmi les acides carboxyliques insaturés, linéaires ou ramifiés.

11. Composition selon la revendication 9, **caractérisée par le fait que** le monomères organique anionique à insaturation éthylénique est choisi, seul ou sous forme de mélange de monomères, parmi l'acide acrylique, l'acide méthacrylique, l'acide maléique, l'anhydride maléique, l'acide itaconique, l'acide fumarique et l'acide crotonique ou leurs sels d'alcalins, d'alcalino-terreux ou d'ammonium, ou leurs mélanges.

12. Composition selon la revendications 9, **caractérisée par le fait que** le monomère organique hydrophobe à insaturation éthylénique est choisi, seul ou en mélange de monomères, parmi les esters d'acide acrylique d' alcanols et/ou les esters d'acide méthacrylique d'alcanols de préférence l'alcanol étant en C₁-C₁₈.

13. Composition selon la revendications 9, **caractérisée par le fait que** le monomère organique hydrophobe à insaturation éthylénique est choisi, seul ou en mélange de monomères dans le groupe constitué par le (méth)acrylate d'isooctyle, le (méth)acrylate d'isonyle, le 2-éthylhexyl(méth)acrylate, le (méth)acrylate de lauryle, le (méth)acrylate d'isopentyle, le (méth)acrylate de n-butyle, le (méth)acrylate d'isobutyle, le (méth)acrylate de méthyle, le (méth)acrylate de tertio-butyle, le (méth)acrylate de tridécyle, le (méth)acrylate de stéaryle.

14. Composition selon l'une quelconque des revendications précédentes, **caractérisée par le fait que** le polymère siliconé greffé comprend sur la chaîne silicone principale, au moins un groupement organique à caractère anionique obtenu par 1'(homo)polymérisation radicalaire d'au moins un monomère anionique de type acide carboxylique insaturé, partiellement ou totalement neutralisé sous la forme d'un sel.

15. Composition selon l'une quelconque des revendications 1 à 5, **caractérisée par le fait que** le polymère siliconé greffé est choisi parmi les polymères siliconés comportant dans leur structure le motif de formule (I) suivant 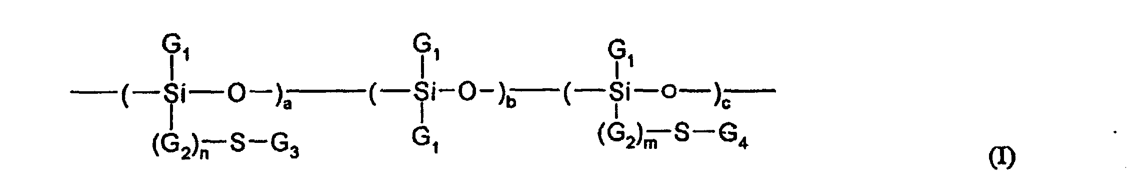 dans lequel les radicaux G₁, identiques ou différents, représentent l'hydrogène ou un radical alkyle en C₁-C₁₀ ou encore un radical phényle ; les radicaux G₂, identiques ou différents, représentent représente un groupe alkylène en C₁-C₁₀; G₃ représente un reste polymérique résultant de l'(homo)polymérisation d'au moins un monomère anionique à insaturation éthylénique ; G₄ représente un reste polymérique résultant de l'(homo)polymérisation d'au moins un monomère hydrophobe à insaturation éthylénique ; m et n sont égaux à 0 ou 1 ; a est un nombre entier allant de 0 et 50 ; b est un nombre entier pouvant être compris entre 10 et 350, c est un nombre entier allant de 0 et 50 ; sous réserve que l'un des paramètres a et c soit différent de 0.

16. Composition selon la revendication 15, **caractérisée par le fait que** le motif de formule (I) présente au moins l'une des caractéristiques suivantes :
- les radicaux G₁ désignent un radical alkyle en C₁-C₁₀;
- n est non nul, et les radicaux G₂ représentent un radical divalent en C₁-C₃;
- G₃ représente un radical polymérique résultant de l'(homo)polymérisation d'au moins un monomère du type acide carboxylique à insaturation éthylénique ;
- G₄ représente un radical polymérique résultant de l'(homo)polymérisation d'au moins un monomère du type (méth)acrylate d'alkyle en C₁-C₁₀.

17. Composition selon la revendication 15 ou 16, **caractérisée par le fait que** le motif de formule (I) présente simultanément les caractéristiques suivantes:
- les radicaux G₁ désignent un radical méthyle;
- n est non nul, et les radicaux G₂ représentent un radical propylène ;
- G₃ représente un radical polymérique résultant de l'(homo)polymérisation d'au moins l'acide acrylique et/ou l'acide méthacrylique ;
- G₄ représente un radical polymérique résultant de l'(homo)polymérisation d'au moins un monomère du type (méth)acrylate d'isobutyle ou de méthyle.

18. Composition selon l'une quelconque des revendications précédentes, **caractérisée par le fait que** la masse moléculaire en nombre du polymère siliconé greffé varie de 10 000 à 1 000 000 environ, et encore plus préférentiellement de 10 000 à 100 000 environ.

19. Composition selon l'une quelconque des revendications précédentes, **caractérisée par le fait qu'**elle comprend, en pourcentage relatif en poids, de 0,1 à 20 %, et de préférence de 0,2 à 10 % de polymère silicone greffé.

20. Composition selon l'une quelconque des revendications précédentes, **caractérisée par le fait qu'**elle contient en outre au moins un additif choisi parmi les tensioactifs anioniques, cationiques, non ioniques ou amphotères, les parfums, les conservateurs, les filtres, les protéines, les vitamines, les polymères autres que ceux de l'invention, les huiles végétales, minérales ou synthétiques.

21. Procédé de maintien ou de mise en forme de la coiffure, **caractérisé par le fait qu'**il comprend la mise en oeuvre d'une composition conforme à l'une quelconque des revendications 1 à 20.

22. Utilisation d'une composition selon l'une quelconque des revendications 1 à 20, pour la fabrication d'un produit cosmétique, en particulier capillaire.

23. Utilisation d'une composition selon la revendication 22 pour les sourcils et les cils.

## Patentansprüche

1. Kosmetische Zusammensetzung, die in einem kosmetisch akzeptablen Medium mindestens ein Silicon/Acrylat-Copolymer und mindestens ein gepfropftes Siliconpolymer mit Polysiloxangerüst, auf das organische nichtsiliconhaltige Monomere gepfropft sind, enthält, **dadurch gekennzeichnet, dass** das Silicon/Acrylat-Copolymer durch radikalische Polymerisation mindestens eines ethylenisch ungesättigten Monomers (a) in Gegenwart mindestens eines Siliconderivats (b) mit Alkylenoxidgruppen hergestellt wird.

2. Zusammensetzung nach Anspruch 1, **dadurch gekennzeichnet, dass** das Monomer (a) der folgenden Formel (Ia) entspricht: 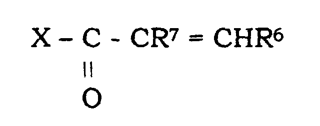 worin:
- X unter OH, OM, OR⁸, NH₂, NHR⁸, N(R⁸)₂ ausgewählt ist;
- M ein Kation bedeutet, das unter Na⁺, K⁺, Mg⁺⁺, NH⁴⁺, Alkylammonium, Dialkylammonium, Trialkylammonium und Tetraalkylammonium ausgewählt ist;
- die Gruppen R⁸ identisch oder voneinander verschieden sein können und unter Wasserstoff, geradkettigen oder verzweigten C₁₋₄₀-Alkylgruppen, mono- oder polyhydroxylierten C₁₋₄₀-Alkylgruppen, die ggf. mit einer oder mehreren Gruppen Alkoxy, Amino oder Carboxy substituiert sind, hydroxylierten Polyethergruppen, N,N-Dimethylaminoethyl, 2-Hydroxyethyl, 2-Methoxyethyl, 2-Ethoxyethyl, Hydroxypropyl, Methoxypropyl und Ethoxypropyl ausgewählt sind;
- die Gruppen R⁷ und R⁶ unabhängig voneinander unter Wasserstoff, geradkettigen oder verzweigten C₁₋₈-Alkylgruppen, Methoxy, Ethoxy, 2-Hydroxyethoxy, 2-Methoxyethoxy et 2-Ethoxyethyl und den Gruppen CN, COOH und COOM ausgewählt sind.

3. Kosmetische Zusammensetzung für das Haar nach Anspruch 1,
**dadurch gekennzeichnet, dass** das Siliconderivat (b) der folgenden Formel (I) entspricht: 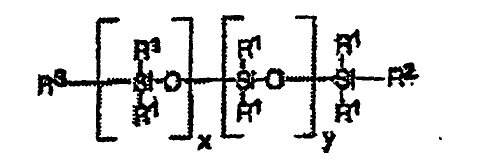 wobei in der Formel (I):
- R² ausgewählt ist unter CH₃ oder der Gruppe 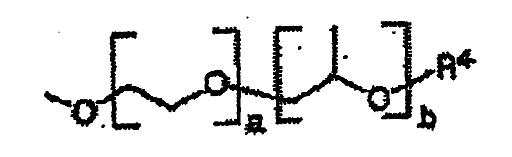
- R³ unter CH₃ oder der Gruppe R² ausgewählt ist,
- R⁴ ausgewählt ist unter Wasserstoff, CH₃ oder den folgenden Gruppen 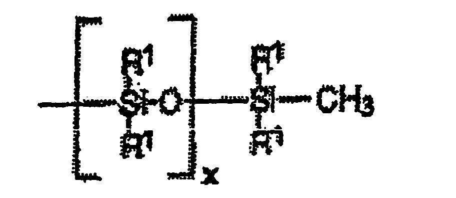 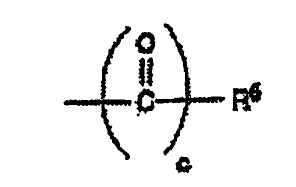
- R⁶ eine organische Gruppe mit 1 bis 40 Kohlenstoffatomen ist, die Amino-, Carboxy- oder Sulfatgruppen enthalten kann, und R₆ das Anion einer anorganischen Säure ist, wenn c=0;
- die Gruppen R¹ identisch oder verschieden sein können und unter den aliphatischen C₁₋₂₀-Kohlenwasserstoffen, aliphatischen oder cycloaliphatischen C₃₋₂₀-Kohlenwasserstoffen und den Gruppen R⁵ der folgenden Formel ausgewählt sind: 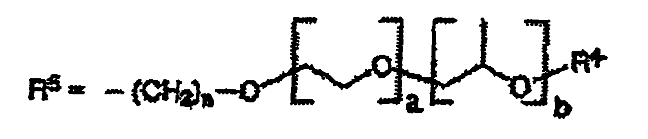
- n eine ganze Zahl im Bereich von 1 bis 6 bedeutet,
- x und y ganze Zahlen sind, die so ausgewählt sind, dass die Molmasse des Polysiloxans im Bereich von 300 bis 30.000 liegt,
- a und b 0 bedeuten oder ganze Zahlen im Bereich von 1 bis 50 sind, und
- c 0 oder 1 bedeutet.

4. Zusammensetzung nach Anspruch 1, **dadurch gekennzeichnet, dass** das Monomer (a) unter den Vinyl- und Allylestern mit 1 bis 40 Kohlenstoffatomen, geradkettigen Carbonsäuren mit 3 bis 40 Kohlenstoffatomen oder cyclischen Carbonsäuren mit 3 bis 40 Kohlenstoffatomen, Vinyl- oder Allylhaliden, Vinyllactamen, vorzugsweise Vinylpyrrolidon und Vinylcaprolactam, mit Vinyl- oder Allylgruppen substituierten heterocyclischen Verbindungen, vorzugsweise Vinylpyridin, Vinyloxazolin und Allylpyridin, N-Vinylimidazolen, Diallylaminen, Vinylidenchlorid, ungesättigten Verbindungen auf Kohlenstoffbasis, wie Styrol oder Isopren, und mit Epichlorhydrin quarternisierten Derivaten von Acrylsäure oder Methacrylsäure ausgewählt ist.

5. Zusammensetzung nach Anspruch 1, **dadurch gekennzeichnet, dass** das Monomer (a) ausgewählt ist unter: Acrylsäure, Methacrylsäure und Ethacrylsäure; Methylacrylat, Ethylacrylat, Propylacrylat, n-Butylacrylat, Isobutylacrylat, *t*-Butylacrylat, 2-Ethylhexylacrylat und Decylacrylat; Methylmethacrylat, Ethylmethacrylat, Propylmethacrylat, n-Butylmethacrylat, Isobutylmethacrylat, t-Butylmethacrylat, 2-Ethylhexylmethacrylat und Decylmethacrylat; Methylethacrylat, Ethylethacrylat, Propylethacrylat, n-Butylethacrylat, Isobutylethacrylat, *t*-Butylethacrylat, 2-Ethylhexylethacrylat und Decylethacrylat; 2,3-Dihydroxypropylacrylat; 2,3-Dihydroxypropylmethacrylat; 2-Dihydroxyethylacrylat; Hydroxypropylacrylat; 2-Hydroxyethylmethacrylat; 2-Hydroxyethylethacryla; 2-Methoxyethylacrylat; 2-Ethoxyethylmethacrylat; 2-Ethoxyethylethacrylat; Hydroxypropylmethacrylat; Glycerylmonoacrylat; Glycerylmonomethacrylat; Polyalkylenglykol(meth)acrylaten, ungesättigten Sulfonsäuren, Acrylamid, Methacrylamid, Ethacrylamid, N,N-Dimethylacrylamid, N-Ethylacrylamid, N-Ethylmethacrylamid, 1-Vinylimidazol, N,N-Dimethylaminoethyl(meth)acrylat, Maleinsäure, Fumarsäure, Maleinsäureanhydrid und ihren Monoestern, Crotonsäure, Itaconsäure, Vinylethern, Vinylformamid, Vinylamin, Vinylpyridin, Vinylimidazol, Vinylfuran, Styrol, Styrolsulfonat, Allylalkohol und deren Gemischen.

6. Zusammensetzung nach einem der vorhergehenden Ansprüche,.
**dadurch gekennzeichnet, dass** die Siliconderivate (b) unter Dimethiconcopolyolen oder siliconhaltigen grenzflächenaktiven Stoffen ausgewählt sind.

7. Zusammensetzung nach einem der vorhergehenden Ansprüche,
**dadurch gekennzeichnet, dass** sie 0,1 bis 20 Gew.-% Silicon/Acrylat-Copolymer und vorzugsweise 0,5 bis 10 Gew.-% Copolymer enthält.

8. Zusammensetzung nach einem der vorhergehenden Ansprüche,
**dadurch gekennzeichnet, dass** das gepfropfte Siliconpolymer eine Polysiloxanhauptkette enthält, auf die im Inneren der Kette sowie ggf. an mindestens einem ihrer Enden mindestens eine organische Gruppe gepfropft ist, die kein Silicon enthält.

9. Zusammensetzung nach einem der vorhergehenden Ansprüche,
**dadurch gekennzeichnet, dass** das gepfropfte Siliconpolymer durch radikalische Copolymerisation mindestens eines nichtsiliconhaltigen anionischen, organischen Monomers mit ethylenischer Doppelbindung und/oder nichtsiliconhaltigen hydrophoben organischen Monomers mit ethylenischer Doppelbindung und eines Polysiloxans, das in der Kette mindestens eine funktionelle Gruppe aufweist, die mit den ethylenischen Doppelbindungen der nichtsiliconhaltigen Monomere reagieren kann, erhältlich ist.

10. Zusammensetzung nach Anspruch 9, **dadurch gekennzeichnet, dass** das anionische organische Monomer mit ethylenischer Doppelbindung einzeln oder in Form von Monomerengemischen unter den geradkettigen oder verzweigten ungesättigten Carbonsäuren ausgewählt ist.

11. Zusammensetzung nach Anspruch 9, **dadurch gekennzeichnet, dass** die anionischen organischen Monomere mit ethylenischer Doppelbindung einzeln oder in Form von Monomerengemischen unter Acrylsäure, Methacrylsäure, Maleinsäure, Maleinsäureanhydrid, Itaconsäure, Fumarsäure und Crotonsäure und deren Alkali-, Erdalkali- oder Ammoniumsalzen oder deren Gemischen ausgewählt sind.

12. Zusammensetzung nach Anspruch 9, **dadurch gekennzeichnet, dass** das hydrophobe organische Monomer mit ethylenischer Doppelbindung einzeln oder in Form von Monomerengemischen unter den Estern von Acrylsäure und Alkanolen und/oder den Estern von Methacrylsäure und Alkanolen ausgewählt ist, wobei das Alkanol vorzugsweise 1 bis 18 Kohlenstoffatome aufweist.

13. Zusammensetzung nach Anspruch 9, **dadurch gekennzeichnet, dass** das hydrophobe organische Monomer mit ethylenischer Doppelbindung einzeln oder in Form von Monomerengemischen unter Isooctyl(meth)acrylat, Isononyl(meth)acrylat, 2-Ethylhexyl(meth)-acrylat, Lauryl(meth)acrylat, Isopentyl(meth)acrylat, n-Butyl(meth)-acrylat, Isobutyl(meth)acrylat, Methyl(meth)acrylat, t-Butyl(meth)-acrylat, Tridecyl(meth)acrylat und Stearyl(meth)acrylat ausgewählt ist.

14. Zusammensetzung nach einem der vorhergehenden Ansprüche,
**dadurch gekennzeichnet, dass** das gepfropfte Siliconpolymer an der Siliconhauptkette mindestens eine organische Gruppe mit anionischem Charakter aufweist, die durch radikalische (Homo)-polymerisation mindestens eines anionischen Monomers vom Typ einer ungesättigten Carbonsäure, die ganz oder teilweise in Form eines Salzes neutralisiert vorliegt, erhalten wird.

15. Zusammensetzung nach einem der Ansprüche 1 bis 5, **dadurch gekennzeichnet, dass** das gepfropfte Siliconpolymer unter den Siliconpolymeren ausgewählt ist, die in ihrer Struktur die Einheit der folgenden Formel (I) aufweist: 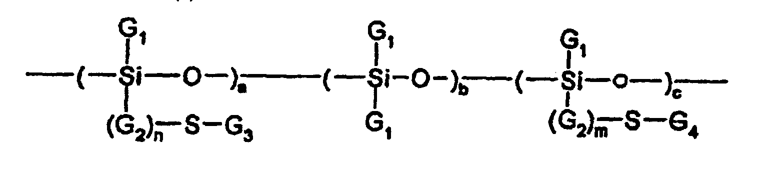 worin die Gruppen G₁, die identisch oder voneinander verschieden sind, Wasserstoff, C₁₋₁₀-Alkyl oder Phenyl; die Gruppen G₂, die identisch oder voneinander verschieden sind, C₁₋₁₀-Alkylen; G₃ eine Polymergruppe, die bei der (Homo)polymerisation mindestens eines anionischen Monomers mit ethylenischer Doppelbindung entsteht; G₄ eine Polymergruppe, die bei der (Homo)polymerisation mindestens eines hydrophoben Monomers mit ethylenischer Doppelbindung gebildet wird; m oder n 0 oder 1; a 0 oder eine ganze Zahl im Bereich von 1 bis 50; b eine ganze Zahl im Bereich von 10 bis 350; und c 0 oder eine ganze Zahl von 1 bis 50 bedeuten; mit der Maßgabe, dass einer der Parameter a oder c von 0 verschieden ist.

16. Zusammensetzung nach Anspruch 15, **dadurch gekennzeichnet, dass** die Einheit (I) mindestens eine der folgenden Eigenschaften aufweist:
- die Gruppen G₁ bedeuten C₁₋₁₀-Alkyl;
- n ist nicht O und die Gruppen G₂ bedeuten eine zweiwertige C₁₋₃-Gruppe;
- G₃ ist eine Polymergruppe, die bei der (Homo)polymerisation mindestens eines Monomers vom Typ einer Carbonsäure mit ethylenischer Doppelbindung entsteht;
- G4 ist eine Polymergruppe, die bei der (Homo)polymerisation mindestens eines Monomers vom Typ C₁₋₁₀-Alkyl(meth)acrylat entsteht.

17. Zusammensetzung nach Anspruch 15 oder 16, **dadurch gekennzeichnet, dass** die Einheit der Formel (I) gleichzeitig die folgenden Eigenschaften aufweist:
- die Gruppen Gi bedeuten Methyl;
- n ist nicht 0 und die Gruppen G₂ bedeuten Propylen;
- G₃ ist eine Polymergruppe, die bei der (Homo)polymerisation von zumindest Acrylsäure und/oder Methacrylsäure entsteht;
- G₄ ist eine Polymergruppe, die bei der (Homo)polymerisation mindestens eines Monomers vom Typ Isobutyl(meth)acrylat oder Methyl(meth)acrylat gebildet wird.

18. Zusammensetzung nach einem der vorhergehenden Ansprüche,
**dadurch gekennzeichnet, dass** das Zahlenmittel der Molmasse des gepfropften Siliconpolymers im Bereich von etwa 10.000 bis 1.000.000 und noch bevorzugter bei 10.000 bis 100.000 liegt.

19. Zusammensetzung nach einem der vorhergehenden Ansprüche,
**dadurch gekennzeichnet, dass** sie 0,1 bis 20 Gew.-% und vorzugsweise 0,2 bis 10 Gew.-% gepfropftes Siliconpolymer enthält.

20. Zusammensetzung nach einem der vorhergehenden Ansprüche,
**dadurch gekennzeichnet, dass** sie ferner mindestens einen Zusatzstoff enthält, der unter den anionischen, kationischen, nichtionischen oder amphoteren grenzflächenaktiven Stoffen, Parfums, Konservierungsmitteln, Filtern, Proteinen, Vitaminen, Polymeren, die von den erfindungsgemäßen Polymeren verschieden sind, und pflanzlichen, mineralischen oder synthetischen Ölen ausgewählt ist.

21. Verfahren zur Festigung oder Formgebung der Frisur, **dadurch gekennzeichnet, dass** eine Zusammensetzung nach einem der Ansprüche 1 bis 20 eingesetzt wird.

22. Verwendung einer Zusammensetzung nach einem der Ansprüche 1 bis 20 zur Herstellung eines kosmetischen Produkts und insbesondere eines kosmetischen Produkts für das Haar.

23. Verwendung einer Zusammensetzung nach Anspruch 22 für Wimpern oder Augenbrauen.

## Claims

1. Cosmetic composition comprising, in a cosmetically acceptable medium, at least one silicone/acrylate copolymer and at least one grafted silicone polymer, containing a polysiloxane skeleton grafted with non-silicone organic monomers, **characterized in that** the silicone/acrylate copolymer is obtained by radical-mediated polymerization of at least one ethylenically unsaturated monomer (a) in the presence of at least one silicone derivative (b) comprising oxyalkylene groups.

2. Composition according to Claim 1, **characterized in that** the monomer (a) corresponds to formula (Iₐ): 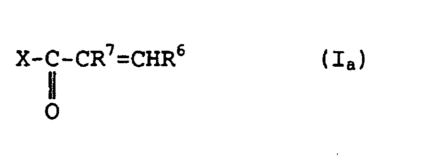 in which:
- X is chosen from the group comprising OH, OM, OR⁸, NH₂, NHR⁸ and N(R⁸)₂;
- M is a cation chosen from Na⁺, K⁺, Mg⁺⁺, NH⁴⁺, alkylammonium, dialkylammonium, trialkylammonium and tetraalkylammonium;
- the radicals R⁸ may be identical or different and are chosen from the group comprising hydrogen, linear or branched C₁ to C₄₀ alkyl groups, mono- or polyhydroxylated C₁ to C₄₀ alkyl groups, optionally substituted with one or more alkoxy, amino or carboxyl groups, hydroxylated polyether groups, and N,N-dimethylaminoethyl, 2-hydroxyethyl, 2-methoxyethyl, 2-ethoxyethyl, hydroxypropyl, methoxypropyl and ethoxypropyl groups;
- R⁷ and R⁶ are chosen, independently of each other, from the group comprising hydrogen, linear or branched C₁ to C₈ alkyl groups, methoxy, ethoxy, 2-hydroxyethoxy, 2-methoxyethoxy and 2-ethoxyethyl groups, and CN, COOH and COOM groups.

3. Cosmetic hair composition according to Claim 1, **characterized in that** the silicone derivative (b) corresponds to the formula (I) below: 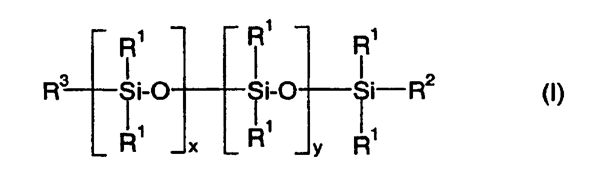 in which formula (I):
- R² is chosen from CH₃ and the group 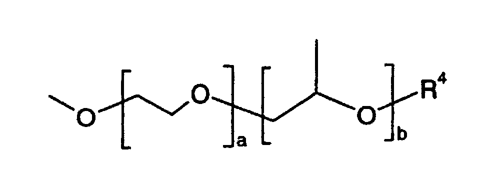
- R³ is chosen from CH₃ and the group R²,
- R⁴ is chosen from hydrogen, CH₃ and the groups 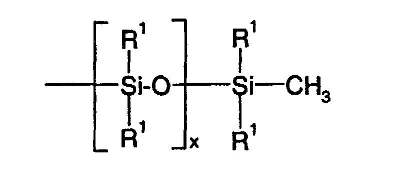 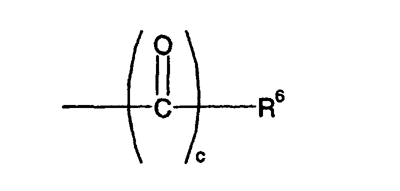
- R⁶ is an organic C₁ to C₄₀ group which can contain amino, carboxyl or sulphonate groups, and if c=0, R₆ is the anion of an inorganic acid;
- the radicals R¹ may be identical or different and are chosen from C₁ to C₂₀ aliphatic hydrocarbons, C₃ to C₂₀ aliphatic or cycloaliphatic hydrocarbons, and the groups R⁵ corresponding to the formula below: 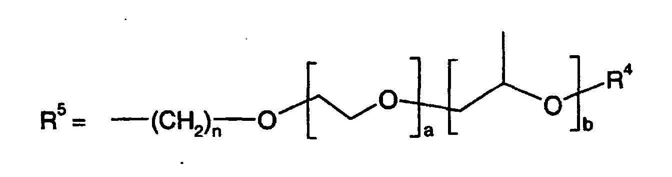
- n is an integer between 1 and 6,
- x and y are integers chosen such that the molecular weight of the polysiloxane is between 300 and 30,000,
- a and b are integers between 0 and 50, and
- c is 0 or 1.

4. Composition according to Claim 1, **characterized in that** the monomer (a) is chosen from C₁ to C₄₀ vinyl and allyl esters, linear C₃ to C₄₀ carboxylic acids or C₃ to C₄₀ carboxycyclic acids, vinyl or allyl halides, vinyllactams, preferably vinylpyrrolidone and vinylcaprolactam, heterocyclic compounds substituted with vinyl or allyl groups, preferably vinylpyridine, vinyloxazoline and allylpyridine, N-vinylimidazoles, diallylamines, vinylidene chloride, carbon-based unsaturated compounds, such as styrene or isoprene, and acrylic or methacrylic acid derivatives quaternized with epichlorohydrin.

5. Composition according to Claim 1, **characterized in that** the monomer (a) is chosen from the group comprising acrylic, methacrylic and ethacrylic acid; methyl, ethyl, propyl, n-butyl, isobutyl, t-butyl, 2-ethylhexyl and decyl acrylate; methyl, ethyl, propyl, n-butyl, isobutyl, t-butyl, 2-ethylhexyl and decyl methacrylate; methyl, ethyl, propyl, n-butyl, isobutyl, t-butyl, 2-ethylhexyl and decyl ethacrylate; 2,3-dihydroxypropyl acrylate; 2,3-dihydroxypropyl methacrylate; 2-dihydroxyethyl acrylate; hydroxypropyl acrylate; 2-hydroxyethyl methacrylate; 2-hydroxyethyl ethacrylate; 2-methoxyethyl acrylate; 2-ethoxyethyl methacrylate; 2-ethoxyethyl ethacrylate; hydroxypropyl methacrylate; glyceryl monoacrylate; glyceryl monomethacrylate; polyalkylene glycol (meth)acrylates, unsaturated sulphonic acids, acrylamide, methacrylamide, ethacrylamide, N,N-dimethylacrylamide, N-ethylacrylamide, N-ethylmethacrylamide, 1-vinylimidazole, N,N-dimethylaminoethyl (meth)acrylate, maleic acid, fumaric acid, maleic anhydride and its monoesters, crotonic acid, itaconic acid, vinyl ethers, vinylformamide, vinylamine, vinylpyridine, vinylimidazole, vinylfuran, styrene, styrene sulphonate and allyl alcohol, and mixtures thereof.

6. Composition according to any one of the preceding claims, **characterized in that** the silicone derivatives (b) are chosen from dimethicone copolyols and silicone surfactants.

7. Composition according to any one of the preceding claims, **characterized in that** it comprises, as a relative percentage by weight, from 0.1% to 20% of silicone/acrylate copolymer, and more preferably from 0.5% to 10% of this copolymer.

8. Composition according to any one of the preceding claims, **characterized in that** the grafted silicone polymer comprises a main polysiloxane chain on which is grafted, inside the said chain as well as, optionally, on at least one of its ends, at least one organic group containing no silicone.

9. Composition according to any one of the preceding claims, **characterized in that** the grafted silicone polymer can be obtained by radical-mediated copolymerization between, on the one hand, at least one non-silicone anionic organic monomer having ethylenic unsaturation and/or a non-silicone hydrophobic organic monomer having ethylenic unsaturation, and, on the other hand, a polysiloxane having in its chain at least one functional group capable of reacting with the said ethylenic unsaturations of the said non-silicone monomers.

10. Composition according to Claim 9, **characterized in that** the anionic organic monomer containing ethylenic unsaturation is chosen, alone or in the form of a mixture of monomers, from linear or branched unsaturated carboxylic acids.

11. Composition according to Claim 9, **characterized in that** the anionic organic monomer containing ethylenic unsaturation is chosen, alone or in the form of a mixture of monomers, from acrylic acid, methacrylic acid, maleic acid, maleic anhydride, itaconic acid, fumaric acid and crotonic acid, or alkali metal, alkaline-earth metal or ammonium salts thereof, or mixtures thereof.

12. Composition according to Claim 9, **characterized in that** the hydrophobic organic monomer containing ethylenic unsaturation is chosen, alone or as a mixture of monomers, from acrylic acid esters of alkanols and/or methacrylic acid esters of alkanols, the alkanol preferably being C₁-C₁₈.

13. Composition according to Claim 9, **characterized in that** the hydrophobic organic monomer containing ethylenic unsaturation is chosen, alone or as a mixture of monomers, from the group consisting of isooctyl (meth)acrylate, isononyl (meth)acrylate, 2-ethylhexyl (meth)acrylate, lauryl (meth)acrylate, isopentyl (meth)acrylate, n-butyl (meth)acrylate, isobutyl (meth)acrylate, methyl (meth)acrylate, tert-butyl (meth)acrylate, tridecyl (meth)acrylate and stearyl (meth)acrylate.

14. Composition according to any one of the preceding claims, **characterized in that** the grafted silicone polymer comprises, on the main silicone chain, at least one organic group of anionic nature obtained by radical-mediated (homo)polymerization of at least one anionic monomer of unsaturated carboxylic acid type, partially or totally neutralized in the form of a salt.

15. Composition according to any one of Claims 1 to 5, **characterized in that** the grafted silicone polymer is chosen from silicone polymers containing in their structure the unit of formula (I) below: 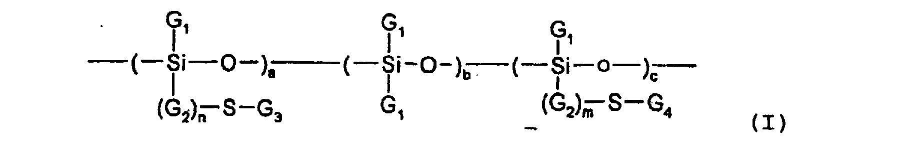 in which the radicals G₁, which may be identical or different, represent hydrogen or a C₁-C₁₀ alkyl radical or alternatively a phenyl radical; the radicals G₂, which may be identical or different, represent a C₁-C₁₀ alkylene group; G₃ represents a polymer residue resulting from the (homo)polymerization of at least one anionic monomer containing ethylenic unsaturation; G₄ represents a polymer residue resulting from the (homo)polymerization of at least one hydrophobic monomer containing ethylenic unsaturation; m and n are equal to 0 or 1; a is an integer ranging from 0 to 50; b is an integer which may be between 10 and 350, c is an integer ranging from 0 to 50; with the proviso that one of the parameters a and c is other than 0.

16. Composition according to Claim 15, **characterized**
**in that** the unit of formula (I) has at least one of the following characteristics:
- the radicals G₁ denote a C₁-C₁₀ alkyl radical;
- n is non-zero and the radicals G₂ represent a divalent C₁-C₃ radical;
- G₃ represents a polymeric radical resulting from the (homo)polymerization of at least one monomer of the carboxylic acid type containing ethylenic unsaturation;
- G₄ represents a polymeric radical resulting from the (homo)polymerization of at least one monomer of the C₁-C₁₀ alkyl (meth)acrylate type.

17. Composition according to Claim 15 or 16,
**characterized in that** the unit of formula (I) simultaneously has the following characteristics:
- the radicals G₁ denote a methyl radical;
- n is non-zero and the radicals G₂ represent a propylene radical;
- G₃ represents a polymeric radical resulting from the (homo)polymerization of at least acrylic acid and/or methacrylic acid;
- G₄ represents a polymeric radical resulting from the (homo)polymerization of at least one monomer of the isobutyl or methyl (meth)acrylate type.

18. Composition according to any one of the preceding claims, **characterized in that** the number-average molecular mass of the grafted silicone polymer ranges approximately from 10,000 to 1,000,000 and even more preferably approximately from 10,000 to 100,000.

19. Composition according to any one of the preceding claims, **characterized in that** it comprises, as a relative percentage by weight, from 0.1% to 20% and preferably from 0.2% to 10% of grafted silicone polymer.

20. Composition according to any one of the preceding claims, **characterized in that** it also contains at least one additive chosen from anionic, cationic, nonionic and amphoteric surfactants, fragrances, preserving agents, screening agents, proteins, vitamins, polymers other than those of the invention, and plant, mineral or synthetic oils.

21. Process for holding or shaping the hairstyle, **characterized in that** it uses a composition in accordance with any one of Claims 1 to 20.

22. Use of a composition according to any one of Claims 1 to 20, for the manufacture of a cosmetic product, in particular a hair product.

23. Use of a composition according to Claim 22 for the eyebrows and the eyelashes.
